# EUROPEAN PATENT APPLICATION

(11) **EP 2 821 420 A1**
(43) Date of publication of application: **07.01.2015**
(21) Application number: 13175124.0
(22) Date of filing: 04.07.2013
(51) Int. Cl.: C08F 283/00, A61Q 5/02, A61Q 5/12, A61K 8/91

(54) **Polymers prepared from alkoxylated polyamines**

(71) Applicant: Akzo Nobel Chemicals International B.V., 3811 MH Amersfoort (NL)
(72) Inventor: Vona Jr., Samuel Anthony, Highland, NY New York 12528 (US); Rodrigues, Klin Aloysius, Signal Mountain, TN Tennessee 37377 (US); Adamo, Anthony John, Flagtown, NJ New Jersey 08821-0215 (US)
(74) Representative: Akzo Nobel IP Department

(57) **Abstract**

An alkoxylated polyamine polymer prepared from an alkoxylated amine and an ethylenically unsaturated monomer containing a free radical polymerizable double bond, as well as formulations, such as personal care formulations, including the alkoxylated polyamine polymer.

## Description

### FIELD OF INVENTION

The invention relates to polymers that are the reaction product of ethylenically unsaturated monomers polymerized with alkoxylated polyamines, and their use in formulations, such as personal care applications.

### BACKGROUND OF THE INVENTION

Cationic polymers are widely used in personal care applications. These cationic polymers are expensive and may have a less than desirable environmental impact. Accordingly, there is a need for polymers that do not suffer from the above disadvantages.

### SUMMARY OF THE INVENTION

The polymers of this invention provide an alternative to the use of pure cationic polymers in personal care formulations and gives formulations improved clarity as well as subjective performance enhancements. In addition, the polymers of this invention may be used for clay soil removal in fabric and cleaning applications.

In an aspect, the invention is directed to a polymer prepared from at least one alkoxylated polyamine and at least one ethylenically unsaturated monomer. In an embodiment, the polymer is polymerized using at least one radical initiator.

In another aspect, the invention is directed to a personal care formulation comprising a polymer and a personal care additive. The polymer is prepared from at least one alkoxylated amine and at least one ethylenically unsaturated monomer containing a free radical polymerizable double bond.

### DETAILED DESCRIPTION OF THE INVENTION

The present invention is generally directed to polymers obtained by reacting one or more ethylenically unsaturated monomers reacted with one or more alkoxylated polyamines using free radical polymerization initiating systems. It has been found that the polymers of the present invention provide similar or improved performance over conventional homopolymers with respect to clarity and deposition in formulations, for example, personal care formulations, such as hair care formulations.

In an embodiment, the polyamines are any materials containing 2 or more amine groups with at least one or more of the amine groups containing a NH group capable of being alkoxylated. Suitable polyamines include, but are not limited to, polyalkyleneamines, polyethyleneimines, poly(alkanolamines), polymers with amine functionality and the like. Examples of polyalkyleneamines include, but are not limited to, ethylenediamine,triethylenetetraamine, tetraethylenepentamine, hexamethylenedamine, bishexamethylenetramine and the like. Examples ofpoly(alkanolamines) include, but are not limited to, condensation products oftriethanolamine, diethanol amine and mixtures thereof. Examples of polymers with amine functionalities are poly(vinyl amine) made from the hydrolysis of poly (vinyl formamide) and poly(vinyl acetamide). Combinations of one or more polyamines may also be used.

In an embodiment, the alkoxylation of the polyamine can be accomplished with any alkylene oxide. Some non-limiting examples of suitable alkylene oxides include ethylene oxide (ethoxylation), propylene oxide (propoxylation), and butylene oxide (butoxylation) and mixtures thereof. In an embodiment, the preferred alkylene oxide is ethylene oxide. In an embodiment, the ethoxylation is at about 10 mole% or more of the alkoxylation, in another embodiment preferably at about 50 mole% or more of the alkoxylation, and in yet another embodiment more preferably at about 80 mole% or more of the alkoxylation and in still yet another embodiment most preferably at 100 mole% of the alkoxylation. The terminal portion of the alkoxylate, preferably an ethoxylate, is an OH group. This OH group may be further functionalized to form a sulfate moiety.

The level of alkoxylation of the polyamine will vary according to the properties desired. In general, the molar ratio of alkoxylation will be from about 1 to about 100 moles of alkylene oxide per mole of polyamine. In an embodiment of this invention, the ratio of alkylene oxide to polyamine will be about 5 to about 60, particularly from about 10 to about 30 moles of alkylene oxide per mole of polyamine. Alternatively stated, in an embodiment, the minimum molar amount of akylene oxide will be greater than about 1, in another embodiment preferably greater than about 5, an in yet another embodiment most preferably greater than about 10 moles of akylene oxide per mole of polyamine. In an embodiment, the maximum level of akylene oxide will be less than about 100, in another embodiment preferably less than about 60, and in yet another embodiment most preferably less than about 30 moles of alkylene oxide per mole of polyamine.

In addition to the alkoxylated polyamines, the polymers of the present invention are obtained from one or more ethylenically unsaturated monomers. The ethylenically unsaturated monomers may be cationic ethylenically unsaturated monomers, anionic ethylenically unsaturated monomers, nonionic ethylenically unsaturated monomers and combinations thereof. As used herein, the term "cationic ethylenically unsaturated monomer" means an ethylenically unsaturated monomer which is capable of introducing a positive charge to the non-anionic copolymer composition. In an embodiment of the present invention, the cationic ethylenically unsaturated monomer has at least one amine functionality. As used herein, "Cationic quaternized ethylenically unsaturated monomer" are those monomers that carry a positive charge at all pH's. "Cationic quaternized ethylenically unsaturated monomer" may be formed by quaternizing a cationic ethylenically unsaturated monomer" to form quaternary ammonium salts, or by oxidizing all or a portion of the amine functionality of a cationic ethylenically unsaturated monomer to form N-oxide groups or forming betaines and sulfobetaines of cationic ethylenically unsaturated monomers.

These cationic ethylenically unsaturated monomers include, but are not limited to, N,N dialkylaminoalkyl(meth)acrylate, N-alkylaminoalkyl(meth)acrylate, N,N dialkylaminoalkyl(meth)acrylamide and N - alkylaminoalkyl(meth)acrylamide, where the alkyl groups are independently C₁₋₁₈, cyclic compounds such as 1-vinyl imidazole and the like. Aromatic amine containing monomers such as vinyl pyridine may also be used. Furthermore, monomers such as vinyl formamide, vinyl acetamide and the like which generate amine moieties on hydrolysis may also be used. Examples of cationic ethylenically unsaturated monomers are N,N-dimethylaminoethyl methacrylate, tert-butylaminoethylmethacrylate and N,N-dimethylaminopropyl methacrylamide. Combinations of cationic ethylenically unsaturated monomers may also be used.

Cationic quarternized ethylenically unsaturated monomers that may be used are diallyldimethylammonium chloride also known as dimethyldiallylammonium chloride, (meth)acrylamidopropyl trimethylammonium chloride, 2-(meth)acryloyloxy ethyl trimethyl ammonium chloride, 2-(meth)acryloyloxy ethyl trimethyl ammonium methyl sulfate, 2-(meth)acryloyloxyethyltrimethyl ammonium chloride, N,N-Dimethylaminoethyl (meth)acrylate methyl chloride quaternary, methacryloyloxy ethyl betaine as well as other betaines and sulfobetaines, 2-(meth)acryloyloxy ethyl dimethyl ammonium hydrochloride, 3-(meth)acryloyloxy ethyl dimethyl ammonium hydroacetate, 2-(meth)acryloyloxy ethyl dimethyl cetyl ammonium chloride, 2-(meth)acryloyloxy ethyl diphenyl ammonium chloride and quarternized derivatives of the N,N dialkylaminoalkyl(meth)acrylate, N-alkylaminoalkyl(meth)acrylate, N,N dialkylaminoalkyl(meth)acrylamide and N - alkylaminoalkyl(meth)acrylamide, such as quaternized derivatives N,N-dimethylaminoethyl methacrylate, tert-butylaminoethylmethacrylate and N,N-dimethylaminopropyl methacrylamide. In an embodiment, monomers are cationic quarternized ethylenically unsaturated monomers. The preferred cationic quarternized ethylenically unsaturated monomers are dimethyldiallylammonium chloride, (meth)acrylamidopropyl trimethylammonium chloride, and N,N-Dimethylaminoethyl (meth)acrylate methyl chloride quaternary.

The polymers prepared using these cationic ethylenically unsaturated monomers or cationic quarternized ethylenically unsaturated monomers are particulary useful in personal care formulations and for clay removal and suspension in cleaning and detergent formulations. In exemplary embodiments these polymers can be used in fabric softener compositions as well as fabric care compositions. Suitable fabric softener formulations contain fabric softener actives, water, surfactants, electrolyte, phase stabilizing polymers, perfume, nonionic surfactant, non-aqueous solvent, silicones, fatty acid, dye, preservatives, optical brighteners, antifoam agents, and mixtures thereof. These fabric softener actives include, but are not limited, to diester quaternary ammonium compounds such as ditallowoyloxyethyl dimethyl ammonium chloride, dihydrogenated-tallowoyloxyethyl dimethyl ammonium chloride, dicanola-oyloxyethyl dimethyl ammonium chloride, ditallow dimethyl ammonium chloride, triethanolamine ester quats such as di-(hydrogenated tallowoyloxyethyl)-N,N-methylhydroxyethylammonium methylsulfate and di-(oleoyloxyethyl)-N, N-methylhydroxyethylammonium methylsulfate as well as others such as tritallow methyl ammonium chloride, methyl bis(tallow amidoethyl)-2-hydroxyethyl ammonium methyl sulfate, methyl bis(hydrogenated tallow amidoethyl)-2-hydroxyethyl ammonium methyl sulfate, methyl bis (oleyl amidoethyl)-2-hydroxyethyl ammonium methyl sulfate, ditallowoyloxyethyl dimethyl ammonium methyl sulfate, dihydrogenated-tallowoyloxyethyl dimethyl ammonium chloride, dicanola-oyloxyethyl dimethyl ammonium chloride, N-tallowoyloxyethyl-N-tallowoylaminopropyl methyl amine, 1,2-bis(hardened tallowoyloxy)- 3-trimethylammonium propane chloride, dihardened tallow dimethyl ammonium chloride and mixtures thereof.

As used herein, the term "anionic ethylenically unsaturated monomer" means an ethylenically unsaturated monomer which is capable of introducing a negative charge to the polymers of this invention. These anionic ethylenically unsaturated monomers can include, but are not limited to, acrylic acid, methacrylic acid, ethacrylic acid, α-chloroacrylic acid, α-cyano acrylic acid, β-methyl-acrylic acid (crotonic acid), α-phenyl acrylic acid, β-acryloxy propionic acid, sorbic acid, α-chloro sorbic acid, angelic acid, cinnamic acid, p-chloro cinnamic acid, β-styryl acrylic acid (1-carboxy-4-phenyl butadiene-1,3), itaconic acid, maleic acid, citraconic acid, mesaconic acid, glutaconic acid, aconitic acid, fumaric acid, tricarboxy ethylene, muconic acid, 2-acryloxypropionic acid, 2-acrylamido-2-methyl propane sulfonic acid, vinyl sulfonic acid, sodium methallyl sulfonate, sulfonated styrene, allyloxybenzene sulfonic acid, vinyl phosphonic acid and maleic acid and their salts. Moieties such as maleic anhydride or acrylamide that can be derivatized (hydrolyzed) to moieties with a negative charge are also suitable. Combinations of anionic ethylenically unsaturated monomers can also be used. In an embodiment of the invention, the anionic ethylenically unsaturated monomer may preferably be acrylic acid, maleic acid, methacrylic acid, itaconic acid, 2-acrylamido-2-methyl propane sulfonic acid or their salts and mixtures thereof.

The polymers of this invention prepared using anionic ethylenically unsaturated monomers are particularly useful for scale control and dispersancy in laundry, automatic dishwash, water treatment, cementing and concrete applications, dispersants in paint/coatings and paper applications and oil field applications. For cleaning applications, the compositions may include, but are not limited to, detergent, fabric cleaner, automatic dishwashing detergent, rinse aids, glass cleaner, fabric care formulation, emulsion breakers, alkaline and acidic hard surface cleaners, laundry detergents and others. These formulations may be liquid, powder, bars or unit dose formulations. The compositions can also be used to clean surfaces in industrial and institutional cleaning applications. In an exemplary embodiment for automatic dishwashing detergent formulations, such formulations include phosphate, low phosphate and "zero" phosphate built formulations, in which the detergent is substantially free of phosphates. As used herein, low phosphate means less than 1500 ppm phosphate in the wash, in another embodiment less than about 1000 ppm phosphate in the wash, and in still another embodiment less that 500 ppm phosphate in the wash.

The polymers of this invention prepared using anionic ethylenically unsaturated monomers can also be used as scale control agents in cleaning, laundry, ADW, oil field, water treatment, and in any other aqueous system where scale buildup is an issue. The scales controlled include, but are not limited to, carbonate, sulfate, phosphate or silicate based scales such as calcium sulfate, barium sulfate, calcium ortho and polyphosphate, tripolyphosphate, magnesium carbonate, magnesium silicate and others.

As used herein, the term "nonionic ethylenically unsaturated monomer" means an ethylenically unsaturated monomer which does not introduce a charge in to the polymers of this invention. These nonionic ethylenically unsaturated monomers include, but are not limited to, acrylamide, methacrylamide, N alkyl(meth)acrylamide, N,N dialkyl(meth)acrylamide such as N,N dimethylacrylamide, hydroxyalkyl(meth)acrylates, alkyl(meth)acrylates such as methylacrylate and methylmethacrylate, vinyl acetate, vinyl morpholine, vinyl pyrrolidone, vinyl caprolactum, ethoxylated alkyl, alkaryl or aryl monomers such as methoxypolyethylene glycol (meth)acrylate, allyl glycidyl ether, allyl alcohol, glycerol (meth)acrylate, monomers containing silane, silanol and siloxane functionalities and others. The preferred nonionic ethylenically unsaturated monomers are acrylamide, methacrylamide, N methyl(meth)acrylamide, N,N dimethyl(meth)acrylamide, vinyl pyrrolidone and vinyl caprolactum, hydroxyethyl (meth)acrylate, and hydroxypropyl (meth)acrylate. Combinations of different nonionic ethylenically unsaturated monomers may also be used.

The polymers of this invention prepared using nonionic ethylenically unsaturated monomers are particularly useful for dispersancy in agricultural applications.

In an embodiment, the ethylenically unsaturated monomers are at least about 1 weight percent, in another embodiment more preferably at least about 2 weight percent and in yet another embodiment most preferably at least about 5 weight percent of the total weight of the monomer and alkoxylated polyamine. In an embodiment, the monomers are at most about 75 weight percent, in another embodiment more preferably at most about 50 weight percent and in yet another embodiment most preferably at most about 25 weight percent of the total weight of the monomer and alkoxylated polyamine.

In preparing the polymers of the present invention, the reaction can be carried out by diluting the alkoxylated polyamine in a solvent. The may be carried out in the molten alkoxylated polyamine provided the monomer and initiator are soluble in this melt. The choice of solvent depends on the monomer, the alkoxylated polyamine and the initiating system. It is preferable that all 3 of these components be soluble in the solvent system at reaction temperature. The preferred solvent is water but if the monomer is hydrophobic an alcohol or glycol based solvent may be used. Mixtures of solvents may be used as needed.

In preparing the polymers of the present invention, the polymers are polymerized using initiators and/or initiating systems. In an embodiment, the initiating system may be any free radical generating system that is soluble in the mixture of the alkoxylated polyamine and the solvent used. In an embodiment, the initiating system is water soluble. Suitable initiators include, but are not limited to peroxides, azo initiators as well as redox systems such as tert-butyl hydroperoxide and erythorbic acid and metal ion based initiating systems. The initiators may include both inorganic and organic peroxides. In an embodiment, the inorganic peroxides, such as sodium persulfate, potassium persulfate and ammonium persulfate are preferred. In another embodiment, the metal ions based initiating systems of Fe and hydrogen peroxide as well as Fe in combination with other peroxides is preferred. In yet another embodiment water soluble azo initiators may be included. Suitable water soluble azo initiator include, but are not limited to, 2,2'-Azobis[2-(2-imidazolin-2-yl)propane]dihydrochloride, 2,2'-Azobis[2-(2-imidazolin-2-yl)propane]disulfate dihydrate, 2,2'-Azobis(2-methylpropionamidine)dihydrochloride, 2,2'-Azobis[N-(2-carboxyethyl)-2-methylpropionamidine]hydrate, 2,2'-Azobis{2-[1-(2-hydroxyethyl)-2-imidazolin-2-yl]propane}dihydrochloride, 2,2'-Azobis[2-(2-imidazolin-2-yl)propane], 2,2'-Azobis(1-imino-1-pyrrolidino-2-ethylpropane)dihydrochloride, 2,2'-Azobis{2-methyl-N-[1,1-bis(hydroxymethyl)-2-hydroxyethl]propionamide}, 2,2'-Azobis[2-methyl-N-(2-hydroxyethyl)propionamide] and others. If solvents are used, then the initiating systems are preferably those soluble in those solvents. Organic peroxide initiators, such as Trigonox® 121 and 421 from AkzoNobel Polymer Chemicals, may be used. In an embodiment, combinations of initiating systems may also be used.

In an embodiment of the invention, the alkoxylated polyamines, such as the ethoxylated polyamines, reacted with cationic quaternized ethylenically unsaturated monomers may be particularly suitable in personal care applications and clay soil removal in laundry applications. In another embodiment, the alkoxylated polyamines, such as ethoxylated polyamines, reacted with anionic ethylenically unsaturated monomers, such as acrylic acid, maleic acid, methacrylic acid, itaconic acid, 2-acrylamido-2-methyl propane sulfonic acid or their salts and mixtures thereof, may be particularly suitable in laundry and dishwash applications. These polymers provide anti-redeposition, anti-encrustation and soil removal in laundry applications and minimize filming and spotting and provide dispersancy and soil suspension in dishwash application. The polymers are good for scale control in water treatment and oil field applications. In yet another embodiment of the invention, the alkoxylated polyamines reacted with nonionic ethylenically unsaturated monomers, such as methyl methacrylate, may be particularly suitable as dispersants in agricultural applications and paints and coating applications, such as water borne paints and coating applications.

In an embodiment of the invention, the polymers may be used in personal care formulations that can include, but are not limited to, formulations for hair styling gels, skin creams, sun tan lotions, moisturizers, tooth pastes, medical and first aid ointments, cosmetic ointments, suppositories, cleansers, lipstick, mascara, hair dye, cream rinse, shampoos, body soap and deodorants, hair care and styling formulations, shave prep and hand sanitizers including alcohol based hand sanitizers.

The personal care formulations may also include formulations for use on the skin, eyelashes or eyebrows, including without limitation, cosmetic compositions such as mascara, facial foundations, eyeliners, lipsticks, and color products; skin care compositions such as moisturizing lotions and creams, skin treatment products, skin protection products in the form of an emulsion, liquid, stick, or a gel; sun care compositions such as sunscreens, sunscreen emulsions, lotions, creams, sunscreen emulsion sprays, liquid/alcohol sunscreen sprays, sunscreen aqueous gels, broad spectrum sunscreens with UVA and UVB actives, sunscreens with organic and inorganic actives, sunscreens with combinations of organic and inorganic actives, suntan products, self-tanning products, and after sun products etc. In an embodiment, suitable compositions are personal care emulsions, and sun care compositions, such as sunscreen emulsions and sunscreen emulsion sprays. The personal care formulation may be in any form, including without limitation in sprays, emulsions, lotions, gels, liquids, sticks, waxes, pastes, powders, and creams.

The personal care formulations may optionally also contain one or more other personal care additive components commonly used in the industry, and these will vary depending upon the type of composition and the functionality and properties desired. Without limitation, these optional other additive components may include, but are not limited to, thickeners, suspending agents, emulsifiers, UV filters, sunscreen actives, humectants, moisturizers, emollients, oils, waxes, solvents, chelating agents, vitamins, antioxidants, botanical extracts, silicones, neutralizing agents, preservatives, fragrances, dyes, pigments, conditioners, polymers, antiperspirant active ingredients, antiacne agents, anti-dandruff actives, surfactants, exfoliants, film formers, propellants, tanning accelerator, hair fixatives and colors. The polymers of the present invention may also be compatible with other components used in conventional personal care formulations. For example, sunscreen formulations may contain at least one additive component selected from the group comprising organic UV filters, inorganic UV actives, UVA and/or UVB suncreen actives, octinoxate, octisalate, oxybenzone, homosalate, octocrylene, avobenzene, titanium dioxide, starch, conditioning agents, emulsifiers, other rheology modifiers and thickeners, neutralizers, emollients, solvents, film formers, moisturizers, antioxidants, vitamins, chelating agents, preservatives, fragrances, and zinc oxide. Skin care and cosmetic formulations may contain at least one personal care additive component selected from the group consisting of vitamins, anti-aging agents, moisturizers, emollients, emulsifiers, surfactants, preservatives, pigments, dyes, colors and insect repellents.

When used in personal care formulations, such as hair care and styling formulations, for example styling gels, optional additive ingredients can be added to provide a variety of additional properties. Various other additives, such as active and functional ingredients, may be included in the personal care formulation as defined herein. These additives may include, but are not limited to, emollients, humectants, thickening agents surfactants, UV light inhibitors, fixative polymers preservatives pigments dyes, colorants, alpha hydroxy acids, aesthetic enhancers such as starch perfumes and fragrances, film formers (water proofing agents) antiseptics, antifungal, antimicrobial and other medicaments and solvents. Additionally, conditioning agents can be used in combination with the polymers of this invention, for example, cationic guar gum, cationic hydroxyethyl cellulose, cationic synthetic polymers and cationic fatty amine derivatives. These blended materials help to provide more substantivity and effective conditioning properties in hair.

Some non-limiting examples of polymers that can used in conjunction with the inventive polymers of this invention include but are not limited to, polyoxythylenated vinyl acetate/crotonic acid copolymers, vinyl acetate crotonic acid (90/10) copolymers, vinyl acetate/crotonic acid/vinyl neodecanoate terpolymers, N-octylacrylamide/methylacrylate/hydroxypropyl methacrylate/acrylic acid/tert-butylaminoethyl methacrylate copolymers, and methyl vinyl ether/maleic anhydride (50/50) copolymers monoesterified with butanol or ethanol, acrylic acid/ethyl acrylate/N-tert-butyl-acrylamide terpolymers, and poly (methacrylic acid/acrylamidomethyl propane sulfonic acid), acrylates copolymer, octylacrylamide/acrylates/butylaminoethyl methacrylate copolymer, acrylates/octylacrylamide copolymer, VA/crotonates/vinyl Neodeanoate copolymer, poly(N-vinyl acetamide), poly(N-vinyl formamide), corn starch modified, sodium polystyrene sulfonate, polyquaterniums such as polyquaternium-4, polyquaternium-7, polyquaternium-10, polyquaternium-11, polyquarternium-16, polyquaternium-28, polyquaternium-29, polyquaternium-46, polyether-1, polyurethanes, VA/acrylates/lauryl methacrylate copolymer, adipic acid/dimethylaminohydroxypropyl diethylene AMP/acrylates copolymer, methacrylol ethyl betaine/acrylates copolymer, PVP/dimethylaminoethylmethacrylate copolymer, PVP/DMAPA acrylates copolymer, PVP/vinylcaprolactam/DMAPA acrylates copolymer, vinyl caprolactam/PVP/dimethylaminoethyl methacrylate copolymer, VA/butyl maleate/isobomyl acrylate copolymer, VA/crotonates copolymer, acrylate/acrylamide copolymer, VA/crotonates/vinyl propionate copolymer, vinylpyrrolidone/vinyl acetate/vinyl propionate terpolymers, VA/crotonates, cationic and amphoteric guar, polyvinylpyrrolidone (PVP), polyvinylpyrrolidone/vinyl acetate copolymer, PVP acrylates copolymer, vinyl acetate/crotonic acid/vinyl proprionate, acrylates/acrylamide, acrylates/octylacrylamide, acrylates/hydroxyacrylates copolymer, and alkyl esters of polyvinylmethylether/maleic anhydride, diglycol/ cyclohexanedimethanol/isophthalates/sulfoisophthalates copolymer, vinyl acetate/butyl maleate and isobornyl acrylate copolymer, vinylcaprolactam/PVP/dimethylaminoethyl methacrylate, vinyl acetate/alkylmaleate half ester/N-substituted acrylamide terpolymers, vinyl caprolactam/vinylpyrrolidone/ methacryloamidopropyl trimethylammonium chloride terpolymer methacrylates/acrylates copolymer/amine salt, polyvinylcaprolactam, polyurethanes, hydroxypropyl guar, hydroxypropyl guar hydroxypropyl trimmonium chloride, poly (methacrylic acid/acrylamidomethyl propane sulfonic acid, poylurethane/acrylate copolymers and hydroxypropyl trimmonium chloride guar, particularly acrylates copolymer, octylacrylamide/acrylates/butylaminoethyl methacrylate copolymer, acrylates/octylacrylamide copolymer, VA/crotonates/vinyl Neodeanoate copolymer, poly(N-vinyl acetamide), poly(N-vinyl formamide), polyurethane, corn starch modified, sodium polystyrene sulfonate, polyquaternium-4, polyquarternium-10, and polyurethane/acrylates copolymer. Combinations of the various personal care additives and polymers may also be included.

In addition to the polymers of this invention, the personal care formulations may also include personal care additives as cosmetically acceptable ingredients. The ingredients can be a emollient, fragrance. exfoliant, medicament, whitening agent, acne treatment agent, a preservative, vitamins, proteins, a cleanser or conditioning agent or a combination thereof.

Examples of cleansers suitable for use the present invention include, but are not limited to, are sodium lauryl sulfate (SLS), sodium laureth sulfate (SLES), ammonium lauryl ether sulfate (ALES), alkanolamides, alkylaryl sulfonates, alky!ary! sulfonic acids, alkylbenzenes, a e acetates, amine oxides, amines, sulfonated amines and amides, betaines, block polymers, carboxylated alcohol or alkylphenol ethoxylates, diphenyl sulfonate derivatives, ethoxylated alcohols, ethoxylated alkylphenols, ethoxylated amines and/or amides, ethoxylated fatty acids, ethoxylated fatty esters and oils, fatty esters (other than glycol, glycerol, etc.), fluorocarbon-based surfactants, glycerol esters, glycol esters, heterocyclics, imidazolines and imidazoline derivatives, isethionates, lanolin-based derivatives, lecithin and lecithin derivatives, lignin and lignin derivatives, methyl esters, monoglycerides and derivatives, olefin sulfonates, phosphate esters, phosphorous organic derivatives, polymeric (polysaccharides, acrylic acid, acrylamide), propoxylated and ethoxylated fatty acids, propoxylated and ethoxylated fatty alcohols, propoxylated and ethoxylated alkyl phenols, protein-based surfactants, quaternary surfactants, sarcosine derivatives, silicone-based surfactants, soaps, sorbitan derivative, sucrose and glucose esters and derivatives, sulfates and sulfonates of oils and fatty acids, sulfates and sulfonates ethoxylated alkyl phenols, sulfates of alcohols, sulfates of ethoxylated alcohols, sulfates of fatty esters, sulfonates of benzene, cumene, toluene and xylene, sulfonates of condensed naphthalenes, sulfonates of dodecyl and tridecyl benzenes, sulfonates of naphthalene and alkyl naphthalene, sulfonates of petroleum, sulfosuccinamates, sulfosuccinates and derivatives.

Preservatives are often used in personal care formulations to provide long term shelf stability. These can be selected from among methylparaben, propylparaben, butylparaben, DMDM hydantoin, imidazolidinyl urea, gluteraldehyde, phenoxyethanol, benzalkonium chloride, methane ammonium chloride, benzethonium chloride, benzyl alcohol, chlorobenzyl alcohol, methylchloroisothiazolinone, methylisothiazolinone, sodium benzoate, chloracetamide, triclosan, iodopropynyl butylcarbamate, sodium pyrithione, and zinc pyrithione. Combinations of these preservatives may also be used.

In an embodiment of this invention, particularly where the hair formulation is a shampoo, the formulation may contain a sulfate free surfactant in addition to the polymers of this invention. Examples of sulfate free surfactants include, but are not limited to, ethoxylated alkylphenols, ethoxylated amines and/or amides, ethoxylated fatty acids, ethoxylated fatty esters and oils, fatty esters (other than glycol, glycerol, etc.), fluorocarbon-based surfactants, glycerol esters, glycol esters, heterocyclics, imidazolines and imidazoline derivatives, isethionates, lanolin-based derivatives, lecithin and lecithin derivatives, lignin and lignin derivatives, methyl esters, monoglycerides and derivatives, phosphate esters, phosphorous organic derivatives, polymeric (polysaccharides, acrylic acid, acrylamide), propoxylated and ethoxylated fatty acids, propoxylated and ethoxylated fatty alcohols, propoxylated and ethoxylated alkyl phenols, protein-based surfactants, quaternary surfactants, sarcosine derivatives, siliconebased surfactants, alpha-olefin sulfonate, alkylaryl sulfonates, sulfonates of oils and fatty acids, sulfonates of ethoxylated alkyl phenols, sulfonates of benzene, cumene, toluene and xylene, sulfonates of condensed naphthalenes, sulfonates of dodecyl and tridecyl benzenes, sulfonates of naphthalene and alkyl naphthalene, sulfonates of petroleum and derivatives thereof. In an embodiment of the invention, the sulfate free surfactants are sulfonates or ethoxylates.

In another embodiment the formulation may include sulfated surfactants. Some non-limiting examples of sulfated surfactants are sodium lauryl sulfate (SLS), sodium laureth sulfate (SLES), alkanolamides, alkylaryl sulfonic acids, sulfates of oils and fatty acids, sulfates of ethoxylated alkyl phenols, sulfates of alcohols, sulfates of ethoxylated alcohols, sulfates of fatty esters, sulfosuccinamates, sulfosuccinates and derivatives thereof.

In addition, shampoo formulations including the polymers of the present invention may optionally include other ingredients. Some non-limiting examples of these ingredients include, but are not limited to, conditioning agents such as silicone oils, either volatile or non-volatile, natural and synthetic oils. Suitable silicone oils that can be added to the compositions include dimethicone, dimethiconol, polydimethylsiloxane, silicone oils with various DC fluid ranges from Dow Coming. Suitable natural oils, such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate can also be used. Some examples of non-ionic conditioning agents are polyols such as glycerin, glycol and derivatives, polyethyleneglycols, which may be known by the trade names Carbowax® PEG from Union Carbide and Polyox® WSR range from Amerchol, polyglycerin, polyethyleneglycol mono- or di- fatty acid esters.

In another embodiment, suitable cationic polymers may be used in the personal care formulation are those best known by their CTFA category name Polyquaternium. Some examples of this class of polymer are Polyquaternium 6, Polyquaternium 7, Polyquaternium 10, Polyquaternium 11, Polyquaternium 16, Polyquaternium 22 and Polyquaternium 28, Polyquaternium 4, Polyquaternium 37, Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

Naturally derived cellulose type polymers known as Polymer JR® type from Amerchol, Polyquaternium 10 or cationic guar gum known with trade name Jaguar® from Rhone-Poulenc, and Guar hydroxypropyl trimonium chloride, chitosan and chitin can also be included in the personal care formulations as cationic natural polymers may also optionally be included with the inventive polymers.

In an embodiment, the polymers of this invention may be included in personal care formulations at least from about 0.1% polymer by weight of the formulation, in another embodiment more preferably at least from about 0.5% polymer by weight of the formulation and in yet another embodiment most preferably at least from about 1.0% polymer by weight of the formulation. In an embodiment, the polymers of this invention may be added to personal care formulations at most from about 20% polymer by weight of the formulation, in another embodiment more preferably at most from about 15% polymer by weight of the formulation and in yet another embodiment most preferably at most from about 10% polymer by weight of the formulation.

The polymers of this invention can also be used in for removal and suspension of clay and other soils in cleaning and detergent compositions that include one or more surfactants, such as those selected from anionic, nonionic, cationic, amphoteric, and zwitterionic surfactants. In an embodiment, the preferred surfactants are suitable for use in these detergent compositions are mixtures of anionic and nonionic surfactants although it is to be understood that any surfactant may be used alone or in combination with any other surfactant or surfactants. Suitable surfactants are generally known by those of ordinary skill in art.

These cleaning and detergent compositions include those formulations for use in home applications as well as those for use in industrial and institutional applications. These formulations may be used to clean clothes, dishes (both hand and automatic dishwash), hard surfaces like bathroom and kitchens and other surfaces.

The polymers of this invention may also be used in cleaning and detergent compositions and may further optionally comprise at least one cleaning and/or detergent additive. Suitable additives may include, for example, builders, dispersants, polymers, ion exchangers, alkalies, anticorrosion materials, antiredeposition materials, antistatic agents, optical brighteners, perfumes, fragrances, dyes, fillers, oils, chelating agents, enzymes, fabric whiteners, brighteners, sudsing control agents, solvents, hydrotropes, bleaching agents, bleach precursors, buffering agents, soil removal agents, soil release agents, fabric softening agents, and opacifiers. In general, such additives and their amounts are known to those of ordinary skill in the art.

In an embodiment, the polymers of this invention may be added to the above-described formulations from at least about 0.1 % polymer by weight of the formulation, in another embodiment more preferably from at least about 0.5% polymer by weight of the formulation and in yet another embodiment most preferably from at least about 1.0% polymer by weight of the formulation. In an embodiment, the polymers of this invention may be added to these cleaning and detergent formulations from at most about 20% polymer by weight of the formulation, in another embodiment more preferably from at most about 15% polymer by weight of the formulation and in yet another embodiment most preferably from at least about 10% polymer by weight of the formulation.

### EXAMPLES

### Preparation of bis(hexamethylene)triamine with 20EO/NH (HMTA-20):

In a 600-mL autoclave was placed 56.0 g of molten bis(hexamethylene)triamine [BHMTA], which was dehydrated by sparging with nitrogen at 110°C for 40 min. Then the amine was treated with 52 g or 5 equivalents of ethylene oxide (EO) at 110° to form BHMTA-5EO or BHMTA with 1EO/NH.

A 4.8-g portion of 45% potassium hydroxide was added, and the mixture was again dehydrated at 110°C for 1 hr before resuming ethylene oxide addition. After 343.6 g of EO had added over 6 hr, the reaction was digested for 1 hr, sparged with nitrogen for 30 min and then cooled to discharge BHMTA-6EO/NH.

A 383.4-g portion of BHMTA-6EO/NH was transferred to a 2-L autoclave where 768.8 g of EO continued addition at 110°C for 3 hr before workup as above and neutralization with 2.2 g of acetic acid. A total of 1147.1 g of BHMTA with 20EO/NH was discharged as a brown oil that solidified on cooling to ambient temperature.

The process described above was also used to synthesize the other ethoxylated polyamines used in the examples that follow.

### Example 1 - Preparation of Sample 1:

The bis(hexamethylene)triamine with 20EO/NH (as described above) was melted by heating to about 40-50°C. 50 grams of this molten material was dissolved in 52 grams of water in a reactor. The amine was neutralized by adding 2.9 grams of 37.6% hydrochloric acid solution. The reaction mixture was heated to 85°C. A monomer feed containing 11.3 grams [3-(Methacryloylamino)propyl]trimethylammonium chloride (MAPTAC), which is a 49% solution in water diluted with 30 grams of water was added over 90 minutes. Simultaneously, an initiator solution containing 0.24 grams of sodium persulfate dissolved in 30 grams of water was added over 90 minutes. The reaction product was held at about 85°C for 1 hour. The final product was an amber solution with 3 1 % solids.

### Example 2 - Preparation of Sample 2:

The bis(hexamethylene)triamine with 20EO/NH was melted by heating to about 40-50°C. 50 grams of this molten material was dissolved in 52 grams of water in a reactor. The amine was neutralized by adding 2.9 grams of 37.6% hydrochloric acid solution. The reaction mixture was heated to about 85°C. A monomer feed containing 34 grams [3-(Methacryloylamino)propyl]trimethylammonium chloride (MAPTAC), which is a 49% solution in water diluted with 30 grams of water was added over 90 minutes. Simultaneously, an initiator solution containing 0.72 grams of sodium persulfate dissolved in 30 grams of water was added over 90 minutes. The reaction product was held at 85C for 1 hour. The final product was an amber solution with 33% solids.

### Example 3 - Preparation of Sample 3:

The bis(hexamethylene)triamine with 20EO/NH was melted by heating to about 40-50°C. 50 grams of this molten material was dissolved in 52 grams of water in a reactor. The amine was neutralized by adding 3.3 grams of 37.6% hydrochloric acid solution. The reaction mixture was heated to 85°C. A monomer feed containing 6.9 grams dimethylaminoethyl methyl chloride quat (DMAEMA quat), which is an 80% solution in water diluted with 30 grams of water was added over 90 minutes. Simultaneously, an initiator solution containing 0.25 grams of sodium persulfate dissolved in 30 grams of water was added over 90 minutes. The reaction product was held at about 85°C for 1 hour. The final product was an amber solution with 33% solids.

### Example 4 - Preparation of Sample 4:

The bis(hexamethylene)triamine with 20EO/NH was melted by heating to about 40-50°C. 50 grams of this molten material was dissolved in 52 grams of water in a reactor. The amine was neutralized by adding 3.3 grams of 37.6% hydrochloric acid solution. The reaction mixture was heated to 85°C. A monomer feed containing 20.8 grams dimethylaminoethyl methyl chloride quat (DMAEMA quat), which is an 80% solution in water diluted with 30 grams of water was added over 90 minutes. Simultaneously, an initiator solution containing 0.76 grams of sodium persulfate dissolved in 30 grams of water was added over 90 minutes. The reaction product was held at about 85°C for 1 hour. The final product was an amber solution with 36.8% solids.

### Example 5 - Preparation of Sample 5:

The bis(hexamethylene)triamine with 20EO/NH was melted by heating to about 40-50°C. 50 grams of this molten material was dissolved in 52 grams of water in a reactor. The amine was neutralized by adding 3.3 grams of 37.6% hydrochloric acid solution. The reaction mixture was heated to about 85°C. A monomer feed containing 11.3 grams [3-(Methacryloylamino)propyl]trimethylammonium chloride (MAPTAC), which is a 49% solution in water diluted with 30 grams of water was added over 90 minutes. Simultaneously, an initiator solution containing 0.24 grams of sodium persulfate dissolved in 30 grams of water was added over 90 minutes. The reaction product was held at about 85°C for 1 hour. The final product was an amber solution with 32.7% solids.

### Example 6 - Preparation of Sample 6:

The bis(hexamethylene)triamine with 20EO/NH was melted by heating to about 40-50°C. 75 grams of this molten material was dissolved in 78 grams of water in a reactor. The amine was neutralized by adding 5 grams of 37.6% hydrochloric acid solution. The reaction mixture was heated to about 85°C. A monomer feed containing 51 grams [3-(Methacryloylamino)propyl]trimethylammonium chloride (MAPTAC), which is a 49% solution in water diluted with 45 grams of water was added over 90 minutes. Simultaneously, an initiator solution containing 1.08 grams of sodium persulfate dissolved in 45 grams of water was added over 90 minutes. The reaction product was held at about 85°C for 1 hour. The final product was an amber solution with 35.7% solids.

### Example 7 - Preparation of a shampoo formulation containing a polymer (lightly conditioning):

A total of 24.69 grams of water was added to a 250ml glass beaker and mixed with a good vortex extending to the bottom of the beaker. Then 0.76 grams of Sample 3(32.9% active) prepared in accordance with Example 3 was added to the beaker. This was mixed for 5 minutes until uniform. The mixing was slowed with the vortex extending 1 inch from the surface of the beaker. This was followed by adding 36.30 grams of Sodium Laureth Sulfate (Standapol® ES-2 from Cognis Corporation, FairField, NJ) and mixed until it was homogenous. Then 21 grams of Sodium Lauryl Sulfate (Witcolate™ WAC LA, Akzo Nobel, Houston , TX) was added and mixed until homogenous. This was followed by adding 14.25 grams of Cocamidoproply Betaine (Crodateric CAB 30, Croda Inc, Edison, NJ) it was allowed to mix until uniform. Then 1 gram of PEG/PPG-15/15 Dimethicone (Dow Coming 5330 Fluid, Dow Coming Corp, Midland, MI) was added and allowed to mix until homogenous. This caused a significant drop in the viscosity of the batch. Once the batch was homogenous, Sodium Chloride (Fisher Scientific, Fairlawn, NJ) was added and the batch was allowed to continue to mix until homogenous. This caused an increase in the viscosity of the batch. The pH was then adjusted to 5.5-6.0 using 10% citric acid (Fisher Scientific, Fairlawn, NJ) and the batch was mixed until homogenous. Then 0.5 grams of DMDM Hydantion (and) Iodopropynyl butylcarbamate (Glydant Plus (Liquid), Lonza Corp Allendale , NJ) was added and the batch was mixed until uniform.

### Example 8 - Preparation of a Shampoo Base Formulation containing polymer blends (lightly conditioning):

A total of 21.25 grams of water was added to a 250ml glass beaker and mixed with a good vortex extending to the bottom of the beaker. Then 0.51 grams Bis(Hexamethylene) Triamine-100E0 (AkzoNobel, Croton River Laboratory NY) was added to the beaker. This was mixed for 5 minutes until uniform. This was followed by adding 3.69 grams of DMAEMA, Ageflex FM1 available from BASF) and the batch was mixed until it was uniform and homogenous. The mixing was slowed with the vortex extending 1 inch from the surface of the beaker. This was followed by adding 36.30 grams of Sodium Laureth Sulfate (Standapol® ES-2 from Cognis Corporation) and mixed until it was homogenous. Then 21 grams of Sodium Lauryl Sulfate (Witcolate™ WAC LA, Akzo Nobel, Houston , TX) was added and mixed until homogenous. This was followed by adding 14.25 grams of Cocamidoproply Betaine (Crodateric CAB 30 , Croda Inc, Edison , NJ) it was allowed to mix until uniform. Then 1 gram of PEG/PPG-15/15 Dimethicone (Dow Coming 5330 Fluid, Dow Coming Corp) was added and allowed to mix until homogenous. This addition caused a significant loss in viscosity to the batch. Once the batch was homogenous Sodium Chloride (Fisher Scientific, Fair lawn, NJ) was added and the batch was allowed to continue to mix until homogenous. This cased the batch to build viscosity. The pH was then adjusted to 5.5-6.0 using 10% citric acid (Fisher Scientific, Fair lawn, NJ) and the batch was mixed until homogenous. Then 0.5 grams of DMDM Hydantion (and) Iodopropynyl butylcarbamate (Glydant Plus (Liquid), Lonza Corp Allendale , NJ) was added and the batch was mixed until homogenous.

**Table 1 - Comparison of copolymers vs. blends of comparable composition**

| Sample | pH | Viscosity cps | Clarity (NTU) |
|---|---|---|---|
| Light Conditioning Shampoo with Sample 3 | 5.84 | 2842 | 14.6 |
| Light Condition Shampoo with HTMA and DMAEMA (Physical Blend) with the same composition as Sample 3 | 5.88 | 3060 | 233 |
| | | | |
| Light Conditioning Shampoo with Sample 4 | 5.95 | 3060 | 15.4 |
| Light Condition Shampoo with HTMA and DMAEMA (Physical Blend) with the same composition as Sample 4 | 5.89 | 3640 | 161 |

| | | | |
|---|---|---|---|
| Unless otherwise stated in the present application, all measurements of viscosity were made using a Brookefield viscometer, RV #5 @10rpm at about 25°C. Unless otherwise stated in the present application, all clarity measurements were made using a Hach 2100N turbidity meter) | | | |

Table 1 shows the much improved clarity of light conditioning shampoos formulated with the polymers of this invention compared to the blends of the homopolymers of DMAEMA and the ethoxylated polyamine (HTMA). The clarity is an indication of the compatibility of the co-polymer with the shampoo formulation. This improved compatibility will result in better stability and improved thickening properties.

### Example 9 - Preparation of a shampoo formulation containing a polymer (non-conditioning) :

A total of 24.69 grams of water was added to a 250ml glass beaker and mixed with a good vortex extending to the bottom of the beaker. Then 0.76 grams of Sample 3 (32.9% active) prepared in accordance with Example 3 was added to the beaker. This was mixed for 5 minutes until uniform. The mixing was slowed with the vortex extending 1 inch from the surface of the beaker. This was followed by adding 36.30 grams of Sodium Laureth Sulfate (Standapol® ES-2 from Cognis Corporation, FairField, NJ) and mixed until it was homogenous. Then 21 grams of Sodium Lauryl Sulfate (Witcolate™ WAC LA, Akzo Nobel, Houston , TX) was added and mixed until homogenous. This was followed by adding 14.25 grams of Cocamidoproply Betaine (Crodateric CAB 30, Croda Inc, Edison, NJ) it was allowed to mix until uniform. Once the batch was homogenous, sodium chloride (Fisher Scientific, Fairlawn, NJ) was added and the batch was allowed to continue to mix until homogenous. This caused an increase in the viscosity of the batch. The pH was then adjusted to 5.5-6.0 using 10% citric acid (Fisher Scientific, Fairlawn, NJ) and the batch was mixed until homogenous. Then 0.5 grams of DMDM Hydantion (and) Iodopropynyl butylcarbamate (Glydant Plus (Liquid), Lonza Corp Allendale , NJ) was added and the batch was mixed until uniform.

### Example 10 - Preparation of a Shampoo Base Formulation containing polymer blends (non-conditioning):

A total of 21.25 grams of water was added to a 250ml glass beaker and mixed with a good vortex extending to the bottom of the beaker. Then 0.51 grams Bis(Hexamethylene) Triamine-100E0 (AkzoNobel, Croton River Laboratory NY) was added to the beaker. This was mixed for 5 minutes until uniform. This was followed by adding 3.69 grams of DMAEMA, Ageflex FM1 available from BASF) and the batch was mixed until it was uniform and homogenous. The mixing was slowed with the vortex extending 1 inch from the surface of the beaker. This was followed by adding 36.30 grams of Sodium Laureth Sulfate (Standapol® ES-2 from Cognis Corporation) and mixed until it was homogenous. Then 21 grams of Sodium Lauryl Sulfate (Witcolate™ WAC LA, Akzo Nobel, Houston , TX) was added and mixed until homogenous. This was followed by adding 14.25 grams of Cocamidoproply Betaine (Crodateric CAB 30 , Croda Inc, Edison , NJ) it was allowed to mix until uniform. Once the batch was homogenous Sodium Chloride (Fisher Scientific, Fair lawn, NJ) was added and the batch was allowed to continue to mix until homogenous. This cased the batch to build viscosity. The pH was then adjusted to 5.5-6.0 using 10% citric acid (Fisher Scientific, Fair lawn, NJ) and the batch was mixed until homogenous. Then 0.5 grams of DMDM Hydantion (and) Iodopropynyl butylcarbamate (Glydant Plus (Liquid), Lonza Corp Allendale , NJ) was added and the batch was mixed until homogenous.

**Table 2 - Comparison of selected polymers versus blends in non-silicon containing shampoos**

| **Conditioning Polymer Sample** # | **pH** | **Viscosity (cPs) (RV** #**5@ 10rpm)** | **Clarity (NTU)** |
|---|---|---|---|
| Sample 4 | 5.97 | 3588 | 165 |
| Sample 5 | 5.72 | 6120 | 141 |
| No Polymer (Blank) | 5.69 | 9804 | 5.1 |
| Control (CELQUAT^{®} SC240) | 5.88 | 8416 | 5.47 |
| Physical Blend of HTMA and DMAEMA) | 5.55 | 2292 | 3306 |

The results shown in Table 2 demonstrate the improved clarity and viscosity compared to samples containing a blend of the cationic polymer and ethoxylated polyamine in the sample concentrations. The formulations containing the copolymer give much improved clarity (compatibility) and thickening properties when compared to the blend in systems that did not contain any silicon oil.

### Lumicrease Dye Test

### SOLUTIONS:

| Stock Dye Solution: | | |
|---|---|---|
| | Pyrazol Fast Red 7BSW | 5.00g |
| | Pylam Products Company, Inc., 2175 East Cedar Street, Tempe, Arizona 85281-7431 | |
| | | |
| | Glacial Acetic Acid | 1.25ml |
| | Deionized Water | dilute to 1000ml in Volumetric Flask |
| | | |

| Test Solution: | | |
|---|---|---|
| | Stock Dye Solution | 100ml |
| | Deionized Water | dilute to 500ml in Volumetric Flask |
| | | |
| Note: Stock Dye Solution should be kept in the refrigerator for no longer than 1 month. | | |

### PROCEDURE:

1) Prepare 200-grams of sample to be tested at 0.50% solids (or obtain neat shampoo product).
2) Label the wool swatches (Test Fabrics - 415 Delaware Ave., West Pittston, PA 18643) in the corner with a permanent marker. Run a duplicate for each sample to be tested.
3) Prepare a 200ml solution of 1% SLES shampoo. Soak the wool swatches in the shampoo solution for 5 minutes while agitating slightly. Remove swatches with tongs and rinse under running water until all the soap is removed.
4) Place each washed and rinsed swatch in a 200ml solution of 0.50% sample to be tested for 1 minute. For finished shampoo system, apply 0.5ml of shampoo on swatch and "work" shampoo into swatch with your hands (use gloves), for 1 minute.
5) Place swatch in a clean 16oz. jar using tongs. Rinse under constant running water for 30 seconds.
6) Pour 100ml of the test solution (Pyrazol dye) into an aluminum hot -melt pan. Place a treated swatch in the test solution for 1 minute. Remove the swatch and rinse again under constant running water, until the water is clear of red dye. Repeat Steps 4 -6 for each swatch.
7) Dry swatches in 110°F oven for 3 hours.
8) After drying, inspect wool swatches for red dye intensity or lack of intensity. The intensity of the red dye indicates how much cationic was left behind on the swatch. The lack of intensity, or the whiteness the swatch, indicates how much of the cationic was rinsed off of the wool swatch.
9) Take photographs of wool swatches to report results.

**Table 3 - Deposition of cationic polymer onto wool**

| 0.5% Active Polymer Solution at pH 5.5-6.0 | Degree of Deposition Based On Color Intensity 1-5 Ranking |
|---|---|
| CELQUAT® SC240 | 2 |
| Sample 1 | 4 |
| Sample 2 | 4.5 |
| Sample 3 | 4 |
| Sample 4 | 4 |
| Sample 6 | 4.5 |
| MAPTAC homopolymer | 4 |

Degree of Deposition Based On Color Intensity was evaluated using a scale of 1-5 based on the intensity of red color. Scoring was conducted based on a value of 5 being the most intense and a value of 1 being the least intense. From the results shown above in table 2, the samples according to the present invention gave the same level of deposition as the homopolymer of MAPTAC and much better deposition than the Celquat® SC 240. The benefit to using the inventive copolymer is the increase viscosity and compatibility shown in Table 1.

### Example 11 - Preparation of Sample 7 (sulfated ethoxylated polyamine):

64.5 grams ofbis(hexamethylene)triamine with 20EO/NH and 40 mole% of the terminal OH groups converted to sulfates (77.5% aqueous solution) was dissolved in 37.6 grams of water in a reactor. The amine was neutralized by adding 3.3 grams of 37.6% hydrochloric acid solution. The reaction mixture was heated to about 85°C. A monomer feed containing 11.3 grams [3-(Methacryloylamino)propyl]trimethylammonium chloride (MAPTAC), which is a 49% solution in water diluted with 30 grams of water was added over 90 minutes. Simultaneously, an initiator solution containing 0.24 grams of sodium persulfate dissolved in 30 grams of water was added over 90 minutes. The reaction product was held at about 85°C for 1 hour. The final product was a yellow color solution with 32.8% solids, pH 6 and the monomer conversion was 95%.

### Example 12 - Preparation of Sample 8 (sulfated ethoxylated polyamine):

64.3 grams ofbis(hexamethylene)triamine with 20EO/NH and 20 mole% of the terminal OH groups converted to sulfates (77.7% aqueous solution) was dissolved in 37.5 grams of water in a reactor. The amine was neutralized by adding 3.3 grams of 37.6% hydrochloric acid solution. The reaction mixture was heated to about 85°C. A monomer feed containing 11.3 grams [3-(Methacryloylamino)propyl]trimethylammonium chloride (MAPTAC), which is a 49% solution in water diluted with 30 grams of water was added over 90 minutes. Simultaneously, an initiator solution containing 0.24 grams of sodium persulfate dissolved in 30 grams of water was added over 90 minutes. The reaction product was held at about 85°C for 1 hour. The final product was a yellow color solution with 33.6% solids, pH 1.6 and the monomer conversion was 96%.

### Example 13 - Preparation of Sample 9 (higher EO content):

The bis(hexamethylene)triamine with 50EO/NH was melted by heating to about 40-50°C. 50 grams of this molten material was dissolved in 48 grams of water in a reactor. The amine was neutralized by adding 3.3 grams of 37.6% hydrochloric acid solution. The reaction mixture was heated to about 85°C. A monomer feed containing 3.8 grams [3-(Methacryloylamino)propyl]trimethylammonium chloride (MAPTAC), which is a 49% solution in water diluted with 30 grams of water was added over 90 minutes. Simultaneously, an initiator solution containing 0.08 grams of sodium persulfate dissolved in 30 grams of water was added over 90 minutes. The reaction product was held at about 85°C for 1 hour. The final product was a yellow solution with 31% solids and a pH or around 1.

### Example 14 - Preparation of Sample 10 (90:10 PEI: AA):

The polyethyleneimine Mw =800 with 20EO/NH was melted by heating to about 40-50°C. 50 grams of this molten material was dissolved in 50 grams of water in a reactor and heated to about 85°C. A monomer feed containing 5.6 grams acrylic acid, diluted with 30 grams of water was added over 30 minutes. Simultaneously, an initiator solution containing 0.74 grams of sodium persulfate dissolved in 30 grams of water was added over 35 minutes. Simultaneously, 6.2 grams of 50% NaOH diluted with 6.2 grams of water was added over 30 minutes. The reaction product was held at about 85°C for 1 hour. The final product was a clear brown solution with 33.5% solids.

### Example 15 - Preparation of sSample 11 (80:10 HMTA: AA):

The bis(hexamethylene)triamine with 20EO/NH was melted by heating to about 40-50°C. 50 grams of this molten material was dissolved in 50 grams of water in a reactor and heated to about 85°C. A monomer feed containing 12.5 grams acrylic acid, diluted with 30 grams of water was added over 30 minutes. Simultaneously, an initiator solution containing 1.65 grams of sodium persulfate dissolved in 30 grams of water was added over 35 minutes. Simultaneously, 13.9 grams of 50% NaOH diluted with 13.9 grams of water was added over 30 minutes. The reaction product was held at about 85°C for 1 hour. The final product was a clear brown solution with 34% solids.

### Example 16 - Preparation of Sample 12 (90:10 PEI: GMAA):

The polyethyleneimine Mw =800 with 20EO/NH was melted by heating to about 40-50°C. 50 grams of this molten material was dissolved in 50 grams of water in a reactor and heated to about 85°C. A monomer feed containing 5.6 grams methacrylic acid, diluted with 30 grams of water was added over 30 minutes. Simultaneously, an initiator solution containing 0.62 grams of sodium persulfate dissolved in 30 grams of water was added over 35 minutes. Simultaneously, 5.2 grams of 50% NaOH diluted with 5.2 grams of water was added over 30 minutes. The reaction product was held at about 85°C for 1 hour. The final product was a clear brown solution with 33.0% solids.

### Example 17 - Preparation of Sample 13 (85:15 HMTA: MMA):

The bis(hexamethylene)triamine with 20EO/NH was melted by heating to about 40-50°C. 50 grams of this molten material was dissolved in 50 grams of water in a reactor and heated to about 85°C. A monomer feed containing 8.5 grams of methylmethacrylate was added over 30 minutes. Simultaneously, an initiator solution containing 0.84 grams of sodium persulfate dissolved in 30 grams of water was added over 35 minutes. The reaction product was held at about 85°C for 1 hour. The final product was opaque yellow with 42% solids.

### Example 18 - Preparation of Sample 14 (HMTA: DMAEMA quat):

The bis(hexamethylene)triamine with 20EO/NH was melted by heating to about 40-50°C. 50 grams of this molten material was dissolved in 50 grams of water in a reactor and heated to about 85°C. A monomer feed containing 6.25 grams of dimethylaminoethyl methacrylate methyl chloride quat 80% was dissolved in 30 grams of water was added over 90 minutes. Simultaneously, an initiator solution containing 0.29 grams of sodium persulfate dissolved in 30 grams of water was added over 35 minutes. The reaction product was held at about 85°C for 1 hour. The final product was a light amber solution with 33% solids.

### Example 19 - Preparation of Sample 15 (HMTA: DMAEMA quat):

The bis(hexamethylene)triamine with 20EO/NH was melted by heating to about 40-50°C. 100 grams of this molten material was heated to about 85°C. A monomer feed containing 12.5 grams of dimethylaminoethyl methacrylate methyl chloride quat 80% was dissolved in 30 grams of water was added over 30 minutes. Simultaneously, an initiator solution containing 0.29 grams of sodium persulfate dissolved in 10 grams of water was added over 35 minutes. The reaction product was held at about 85°C for 1 hour. The final reaction product was diluted with 100 grams of water. The final product was a light amber solution with 47% solids and a pH of 9.3.

### Evaluation for clay soil release:

The polymer of Sample 14 (Example 18) and Sample 15 (Example 19) were evaluated for clay soil removal in Liquid Wisk in a terg-o-tometer. The test used polyester swatches that were stained with Georgia clay. The temperature was 93°C and the water hardness was 150 ppm with a 2:1 ratio of Calcium to Magnesium. The test used a 10 minute wash cycle and 5 minute rinse cycle and the liquid Wisk was dosed at 0.8 grams/liter. The polymer was dosed at 3 active percent of the detergent. The swatches were scanned before and after the wash and the images analyzed to give a delta whiteness index. The higher the delta whiteness index the better the clay soil removal performance.

**Table 4 - Clay soil removal in detergent applications**

| Polymer | Delta whiteness index |
|---|---|
| No polymer | 110 |
| Sample 14 | 182 |
| Sample 15 | 191 |

These data in Table 4 show the polymers of this invention are good clay soil removal polymers in detergent applications as determined by the whiteness index.

All documents cited in the Detailed Description of the Invention are, in relevant part, incorporated herein by reference; the citation of any document is not to be construed as an admission that it is prior art with respect to the present invention.

While particular embodiments of the present invention have been illustrated and described herein, the invention is not intended to be limited to the details shown. Rather, various modifications may be made in the details within the range and scope of equivalents of the claims and without departing from the spirit and scope of the invention.

## Claims

1. A personal care formulation comprising:
an alkoxylated polyamine polymer prepared from at least one alkoxylated amine and at least one ethylenically unsaturated monomer containing a free radical polymerizable double bond; and
a personal care additive.

2. The personal care formulation of claim 1 wherein the at least one alkoxylated polyamine polymer has a level of alkoxylation from about 1 to about 100 moles of alkylene oxide per mole of polyamine.

3. The personal care composition of claim 1 or 2 wherein the at least one alkoxylated amine is prepared from an alkylene oxide selected from the group consisting of ethylene oxide, propylene oxide, butylene oxide and mixtures thereof.

4. The personal care formulation of claim 3 wherein the alkylene oxide is an ethoxylated amine.

5. The personal care formulation of claim 4 wherein ethoxylation is at about 10 mole% or more of the alkoxylation.

6. The personal care formulation of claims 1 to 5 wherein the ethylenically unsaturated monomer is cationic, anionic or nonionic.

7. The personal care formulation of claims 1 to 6 wherein the at least one ethylenically unsaturated monomer is cationic and selected from the group consisting ofN,N dialkylaminoalkyl(meth)acrylate, N-alkylaminoalkyl(meth)acrylate, N,N dialkylaminoalkyl(meth)acrylamide and N - alkylaminoalkyl(meth)acrylamide, where the alkyl groups are independently C₁₋₁₈, cyclic compounds, aromatic amine-containing monomers, vinyl formamide, vinyl acetamide and cationic quarternized ethylenically unsaturated monomers.

8. The personal care formulation of claim 7 wherein the cationic quaternized unsaturated monomer is selected from the group consisting of diallyldimethylammonium chloride, (meth)acrylamidopropyl trimethylammonium chloride, 2-(meth)acryloyloxy ethyl trimethyl ammonium chloride, 2-(meth)acryloyloxy ethyl trimethyl ammonium methyl sulfate, 2-(meth)acryloyloxyethyltrimethyl ammonium chloride, N,N-Dimethylaminoethyl (meth)acrylate methyl chloride quaternary, methacryloyloxy ethyl betaine, 2-(meth)acryloyloxy ethyl dimethyl ammonium hydrochloride, 3-(meth)acryloyloxy ethyl dimethyl ammonium hydroacetate, 2-(meth)acryloyloxy ethyl dimethyl cetyl ammonium chloride, 2-(meth)acryloyloxy ethyl diphenyl ammonium chloride and quarternized derivatives of the N,N dialkylaminoalkyl(meth)acrylate, N-alkylaminoalkyl(meth)acrylate, N,N dialkylaminoalkyl(meth)acrylamide and N - alkylaminoalkyl(meth)acrylamide.

9. The personal care formulation of claim 1 to 8 wherein the personal care formulation is selected from the group consisting of hair styling gels, skin creams, sun tan lotions, moisturizers, tooth pastes, medical and first aid ointments, cosmetic ointments, suppositories, cleansers, lipstick, mascara, hair dye, cream rinse, shampoos, body soap, deodorants, hair care formulations, styling formulations, shave prep, hand sanitizers, facial foundations, eyeliners, color products, sunscreens, lotions, creams, suntan products, after sun products and combinations thereof.

10. The personal care formulations of claim 1 to 9 wherein the personal care additive is selected from the group consisting of thickeners, suspending agents, emulsifiers, UV filters, sunscreen actives, humectants, moisturizers, emollients, oils, waxes, solvents, chelating agents, vitamins, antioxidants, botanical extracts, silicones, neutralizing agents, preservatives, fragrances, dyes, pigments, conditioners, polymers, antiperspirant active ingredients, anti-acne agents, anti-dandruff actives, surfactants, exfoliants, film formers, propellants, tanning accelerator, hair fixatives, colors and combinations thereof.

11. The personal care formulation of claims 1 to 10 wherein the alkoxylated polyamine polymer is present from about 0.1% polymer or more and at most about 20% polymer by weight, based on the weight of the personal care formulation.

12. An alkoxylated polyamine polymer comprising a polymer prepared from at least one alkoxylated amine and at least one ethylenically unsaturated monomer containing a free radical polymerizable double bond.

13. The polymer of claim 12 wherein the alkoxylated polyamine amine is an ethoxylated amine.

14. The polymer of claims 12 or 13 wherein the ethylenically unsaturated monomer is cationic, anionic or nonionic.

15. The polymer of claims 12 to 14 wherein the polymer is prepared by polymerizing the at least one alkoxylated polyamine and the at least one ethylenically unsaturated monomer with an initiator, wherein the radical initiator is selected from the group consisting of peroxides, azo initiators, redox systems, and metal ion based initiating systems.
